(19) Europäisches Patentamt
European Patent Office
Office européen des brevets

(11) EP 2 368 493 B1

(12) FASCICULE DE BREVET EUROPEEN

(45) Date de publication et mention de la délivrance du brevet:
**23.05.2012 Bulletin 2012/21**

(51) Int Cl.:
*A61B 5/04* *(2006.01)*    *G06K 9/00* *(2006.01)*

(21) Numéro de dépôt: **11151353.7**

(22) Date de dépôt: **19.01.2011**

(54) **Dispositif médical actif comprenant des moyens de détection et de filtrage d'artefacts par analyse du vectogramme cardiaque**

Aktive medizinische Vorrichtung mit Erfassungs- und Filtermitteln für Artefakte durch Analyse eines Vektor-Kardiogramms

Active medical device including means for detecting and filtering artefacts by cardiac vector diagram analysis

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **22.03.2010 FR 1052041**

(43) Date de publication de la demande:
**28.09.2011 Bulletin 2011/39**

(73) Titulaire: **Sorin CRM SAS**
**92143 Clamart Cedex (FR)**

(72) Inventeurs:
• **Milpied, Paola**
**75014, PARIS (FR)**
• **Henry, Christine**
**75014, PARIS (FR)**

(74) Mandataire: **Dupuis-Latour, Dominique**
**Bardehle Pagenberg**
**10, boulevard Haussmann**
**75009 Paris (FR)**

(56) Documents cités:
**EP-A1- 2 105 843    US-A1- 2006 235 476**

**Description**

L'invention concerne les "dispositifs médicaux implantables actifs" tels que définis par la directive 90/385/CEE du 20 juin 1990 du Conseil des communautés européennes, plus précisément les implants permettant de surveiller en continu le rythme cardiaque et délivrer si nécessaire au coeur des impulsions électriques de stimulation, de resynchronisation, de cardioversion et/ou de défibrillation en cas de trouble du rythme détecté par le dispositif.

On notera cependant que l'invention peut être mise en oeuvre non seulement au sein d'un implant, mais également à l'extérieur du corps du patient, par exemple dans un programmateur externe utilisé par un praticien pour télécharger et analyser les signaux cardiaques recueillis et mémorisés par l'implant. L'invention peut également être mise en oeuvre dans un moniteur de *home monitoring,* qui est un type particulier de programmateur dont le fonctionnement est entièrement automatisé ; un tel moniteur ne nécessite pas le recours à un praticien, et permet notamment de télé-transmettre à intervalles réguliers à un site distant des données recueillies par un implant, pour analyse et suivi du patient. L'invention peut être également mise en oeuvre au niveau du serveur de données de ce site, à partir des données transmises telles quelles par le moniteur du patient. L'invention est tout particulièrement applicable, quoique de façon non limitative, aux appareils permettant de délivrer au coeur des thérapies antitachycardiques impliquant l'application contrôlée de chocs de défibrillation (impulsions électriques de haute énergie destinées à mettre fin à une tachyarythmie), et/ou de thérapies par stimulation à haute fréquence de type dit ATP (*AntiTachycardia Pacing*).

En effet, ces dispositifs sont sensibles à la détection de signaux d'origine non cardiaque dus à des problèmes liés à la sonde, à des interférences électromagnétiques, à la détection de myopotentiels, etc., phénomènes ci-après désignés sous le terme générique de "bruit".

Ces phénomènes sont susceptibles de générer des artefacts dont la détection par le dispositif peut avoir des conséquences très graves, par exemple en inhibant à tort des stimulations antibradycardiques ou des thérapies de resynchronisation ou bien, inversement, en permettant la délivrance de chocs de défibrillation inappropriés.

En particulier, dans le cas des défibrillateurs implantables, il a été observé par Schulte B et al., Inappropriate Arrhythmia Detection in Implantable Defibrillator Therapy Due to Oversensing of Diaphragmatic Myopotentials, J Interv Card Electrophysiol, 2001. 5(4): p. 487-93, que, dans un groupe de 384 patients implantés d'un défibrillateur, 139 épisodes d'arythmie ont été identifiés à tort comme fibrillation ventriculaire du fait de détection de myopotentiels chez 33 patients (8,6 %), et 32 chocs inappropriés ont été délivrés chez 11 patients (2,9 %). De même, Occhetta E et al., Inappropriate Implantable Cardioverter-Defibrillator Discharges Unrelated to Supraventricular Tachyarrhythmias, Europace, 2006. 8(10): p. 863-9, affirment que, de façon générale, 4 % des patients implantés d'un défibrillateur reçoivent au moins un choc inapproprié du fait de la "sur-détection" de signaux extracardiaques sans aucune relation avec des arythmies supraventriculaires.

Or l'application d'un choc de défibrillation chez un patient conscient est extrêmement douloureuse et angoissante, les énergies appliquées étant en effet très au-delà du seuil de la douleur. En outre, l'application d'un choc de défibrillation n'est pas dénuée d'effets secondaires sur le rythme cardiaque (risque d'apparition de troubles secondaires), sur l'intégrité fonctionnelle du myocarde, et de façon générale sur l'équilibre physiologique du patient.

Il est donc important de ne délivrer de tels chocs que de façon appropriée et seulement si une autre thérapie moins douloureuse, telle qu'une stimulation appropriée de l'oreillette, n'est pas envisageable.

Les procédés les plus utilisés pour s'affranchir des phénomènes de bruit dans la détection des signaux ventriculaires comprennent des étapes de filtrage des signaux recueillis et des contrôles automatiques de différents paramètres de détection tels que période de blanking, valeur de sensibilité, etc. permettant de repérer les sur-détections et interdire dans ce cas toute action inappropriée.

Le US 6 584 351 B1 propose une manière différente de procéder, en tentant d'éliminer le bruit *a priori,* avant la détection ventriculaire. À cet effet, une électrode supplémentaire, implantée hors du coeur, permet de détecter les signaux d'origine non cardiaque, qui seront considérés comme constitutifs de bruit. Ces signaux seront soustraits des signaux détectés par les électrodes intracardiaques. Cette méthode, si elle est efficace, nécessite toutefois l'implantation d'une électrode supplémentaire, hors du coeur, par exemple sur le boîtier du défibrillateur - ce qui implique de revoir complètement la conception mécanique et électrique de celui-ci. D'autres méthodes ont été proposées pour opérer un filtrage *a posteriori* des signaux de bruit extracardiaque contenus dans le signal ventriculaire après recueil de celui-ci.

Le US 2006/0235476 A1 propose ainsi de comparer la chaîne de marqueurs ventriculaires produits par le défibrillateur à partir d'un signal bipolaire ou *nearfield,* avec une chaîne de marqueurs R obtenus par analyse d'un signal unipolaire ou *farfield* recueilli simultanément. Les marqueurs détectés par le défibrillateur à partir du signal *nearfield* mais qui ne sont pas détectés sur le signal *farfield* seront considérés comme étant du bruit, et l'origine de ce bruit sera ensuite déterminée par analyse du nombre et du motif (*pattern*) de la chaîne de marqueurs. Cette technique présente cependant l'inconvénient que, si le signal d'origine extra-cardiaque est présent sur les deux voies (*nearfield* et *farfield*), il ne sera pas considéré comme étant du bruit, et ceci même si ce phénomène est clairement identifiable à l'oeil nu à l'examen des tracés.

Une autre technique encore est proposée par le US 7 215 993 B2, qui propose de calculer une amplitude moyenne pondérée et normalisée à partir : de l'amplitude du battement analysé, de l'amplitude du battement précédent et de l'amplitude du battement suivant. Si la moyenne dépasse un seuil prédéterminé, alors le battement analysé est validé, sinon il est considéré comme étant du bruit. Cette manière de procéder reste cependant sensible à diverses anomalies du rythme survenant de manière erratique, qui sont susceptibles de leurrer l'algorithme et d'entraîner aussi bien des faux positifs que des faux négatifs.

L'idée de base de l'invention réside dans la constatation de ce qu'il est possible d'obtenir des paramètres pertinents relatifs à l'origine du signal recueilli - onde de dépolarisation cardiaque, ou bien autre potentiel générateur d'artefact - par l'analyse de signaux d'électrogramme endocavitaire (signaux EGM) recueillis concurremment sur deux voies distinctes et provenant d'une même cavité, notamment du ventricule.

Les deux voies EGM différentes peuvent en particulier être celle d'un signal unipolaire (signal lointain recueilli entre le boîtier et une électrode distale ou bien proximale), et celle d'un signal bipolaire (signal proche recueilli entre une électrode distale et une électrode proximale).

De façon caractéristique de l'invention, l'analyse de ces signaux est une analyse bidimensionnelle à partir de la "boucle cardiaque" ou "vectogramme" (VGM), qui est la représentation dans un espace à deux dimensions de l'un de ces deux signaux par rapport à l'autre. Cet espace est typiquement un espace "voie unipolaire (en ordonnée) vs. voie bipolaire (en abscisse)", chaque battement ou fraction significative de battement étant alors représenté par son vectogramme dans le plan ainsi défini - en faisant donc abstraction de la dimension temporelle.

On soulignera que ce "vectogramme" (VGM), qui est obtenu à partir de signaux d'électrogramme (EGM) issus de sondes intracardiaques, ne doit pas être confondu avec le "vectocardiogramme" (VCG) qui est, lui, obtenu à partir de signaux d'électrocardiogramme (ECG) issus d'électrodes externes placées sur le thorax du patient.

Une technique apparentée est décrite dans le EP 2 105 843 A1 (ELA Medical), où il est proposé de construire un VGM représentatif du cycle courant à analyser en zone de tachycardie, et de comparer ce VGM courant à un (ou plusieurs) VGM de référence préalablement obtenu en rythme sinusal (RS). Le résultat de cette comparaison permet de classifier le cycle courant à analyser dans l'une des catégories : tachycardie ventriculaire (TV) ou tachycardie supraventriculaire (TSV), en fonction de la proximité plus ou moins grande du VGM courant analysé avec le VGM de référence préalablement acquis et conservé en mémoire.

Dans le cas de la présente invention, comme on le verra plus bas, il ne s'agit toutefois pas de comparer un VGM courant à un ou plusieurs VGM de référence, mais de décrire un vectogramme par un paramètre descripteur de la morphologie intrinsèque du VGM courant. L'analyse de ce descripteur morphologique devra permettre de déterminer ensuite si le battement courant est d'origine cardiaque, ou s'il s'agit de bruit (le paramètre intrinsèque étant par exemple l'angle moyen que forment ies vecteurs vitesse consécutifs le long du parcours de la boucle du VGM).

On notera par ailleurs que l'analyse 'bidimensionnelle" ou "en deux dimensions" (2D) évoquée ici ne doit pas être entendue de manière en elle-même limitative. L'invention peut en effet s'appliquer aussi bien à une analyse dans un espace multidimensionnel d'ordre supérieur (3D ou plus), par extrapolation des enseignements de la présente description à une situation où des signaux EGM provenant d'une même cavité sont recueillis simultanément sur trois voies ou plus.

Plus précisément, l'invention concerne un dispositif médical actif d'un type connu d'après le US 2006/0235476 A1, comprenant : des moyens de recueil de l'activité électrique du coeur sur une série de cycles cardiaques, comprenant des moyens pour produire au moins deux composantes temporelles distinctes à partir d'au moins deux signaux EGM d'électrogramme endocavitaire provenant d'une même cavité du coeur et recueillis concurremment sur des voies respectives distinctes ; et des moyens d'analyse et de filtrage de l'activité électrique recueillie, aptes à discriminer, pour chaque cycle cardiaque analysé, entre cycle bruité invalide et cycle non bruité valide.

De façon caractéristique de l'invention, les moyens d'analyse et de filtrage de ce dispositif comprennent : des moyens pour déterminer une caractéristique 2D non-temporelle représentative du cycle cardiaque analysé, à partir des variations d'une desdites composantes temporelles en fonction d'une l'autre ; des moyens d'analyse morphologique de cette caractéristique 2D, aptes à délivrer au moins un paramètre descripteur intrinsèque de cette caractéristique ; et des moyens classifieurs, aptes à classer le cycle cardiaque analysé comme cycle bruité invalide ou bien comme cycle non bruité valide, en fonction dudit au moins un paramètre descripteur. Les signaux EGM recueillis concurremment sur des voies respectives distinctes peuvent notamment comprendre un signal de composante unipolaire recueilli entre le boîtier du dispositif et une électrode proximale ou distale, et un signal de composante bipolaire recueilli entre une électrode proximale et une électrode distale.

De préférence, les moyens pour déterminer la caractéristique 2D sont des moyens aptes à déterminer cette caractéristique à partir des variations des composantes temporelles sur une fraction du cycle cardiaque analysé, considérée dans une fenêtre temporelle incluant le complexe QRS de ce cycle cardiaque.

Le paramètre descripteur peut notamment être un paramètre fonction du vecteur vitesse de la caractéristique 2D, considéré en une pluralité de points.

Dans une première mise en oeuvre, ce paramètre descripteur est fonction des variations de la direction du vecteur vitesse entre des points consécutifs donnés de la caractéristique 2D, notamment fonction de l'angle moyen que forment deux vecteurs vitesse consécutifs de la caractéristique 2D. Les moyens classifieurs comprennent alors des moyens pour comparer l'angle moyen à un premier seuil donné, et classer le cycle cardiaque à analyser comme cycle bruité invalide si l'angle moyen est supérieur à ce premier seuil. De préférence, si l'angle moyen est inférieur au premier seuil, les moyens classifieurs comparent l'angle moyen à un deuxième seuil donné inférieur au premier seuil, comparent l'amplitude du pic de dépolarisation du cycle cardiaque analysé à un troisième seuil, et classent le cycle cardiaque à analyser comme cycle bruité invalide si l'angle moyen est supérieur au deuxième seuil et l'amplitude du pic de dépolarisation inférieure au troisième seuil, et comme cycle non bruité dans le cas contraire.

Dans une deuxième mise en oeuvre, le paramètre descripteur est fonction de la variance de la norme du vecteur vitesse entre des points consécutifs donnés de la caractéristique 2D.

Très avantageusement, le dispositif comprend en outre des moyens opérant en réponse aux moyens classifieurs pour, lorsque le cycle analysé est classé comme cycle bruité invalide par les moyens classifieurs : effacer un marqueur lié au cycle cardiaque analysé, et/ou attribuer un marqueur spécifique au cycle cardiaque analysé, et/ou inhiber la mémorisation du cycle cardiaque analysé, et/ou modifier la sensibilité des moyens de recueil, et/ou inhiber la délivrance d'une thérapie par le dispositif.

On va maintenant décrire un exemple de mise en oeuvre du dispositif de l'invention, en référence aux dessins annexés où les mêmes références numériques désignent d'une figure à l'autre des éléments identiques ou fonctionnellement semblables.

La Figure 1 est une vue générale montrant une sonde bipolaire implantée au fond du ventricule.

La Figure 2 illustre les signaux EGM obtenus respectivement sur les voies bipolaire ventriculaire et unipolaire ventriculaire de la configuration de la Figure 1.

La Figure 3 illustre la manière dont sont analysés les signaux d'EGM simultanément recueillis sur les deux voies unipolaire et bipolaire, avec fenêtrage autour de l'onde R détectée sur le signal bipolaire, pour huit battements successifs.

La Figure 4 illustre les vectogrammes obtenus en combinant les deux signaux de la Figure 3, pour les divers battements successifs.

La Figure 5 est un organigramme décrivant les différentes étapes de l'algorithme de filtrage selon l'invention.

La Figure 6 est homologue de la Figure 5, pour une version perfectionnée de l'algorithme de filtrage.

La Figure 7 illustre divers exemples de vectogrammes analysés par la technique de l'invention et classés comme bruit.

La Figure 8 illustre divers exemples de vectogrammes analysés par la technique de l'invention et classés comme signaux valides de fibrillation ventriculaire.

On va maintenant décrire un exemple de réalisation du dispositif de l'invention.

En ce qui concerne ses aspects logiciels, l'invention peut être mise en oeuvre par une programmation appropriée du logiciel de commande d'un stimulateur connu, par exemple de type stimulateur cardiaque, resynchroniseur et/ou défibrillateur, comprenant des moyens d'acquisition d'un signal fourni par des sondes endocavitaires et/ou un ou plusieurs capteurs implantés.

L'invention peut notamment être appliquée aux dispositifs implantabies tels que ceux des familles *Reply, Paradym, Ovatio, Esprit* ou *Rhapsody* produits et commercialisés par Sorin CRM SAS, Clamart, France.

Il s'agit de dispositifs à microprocesseur programmable comportant des circuits pour recevoir, mettre en forme et traiter des signaux électriques recueillis par des électrodes implantées, et délivrer des impulsions de stimulation à ces électrodes. Il est possible d'y transmettre par télémétrie des logiciels qui seront conservés en mémoire et exécutés pour mettre en oeuvre les fonctions de l'invention qui seront décrites ci-dessous. L'adaptation de ces appareils à la mise en oeuvre des fonctions de l'invention est à la portée de l'homme du métier, et elle ne sera pas décrite en détail.

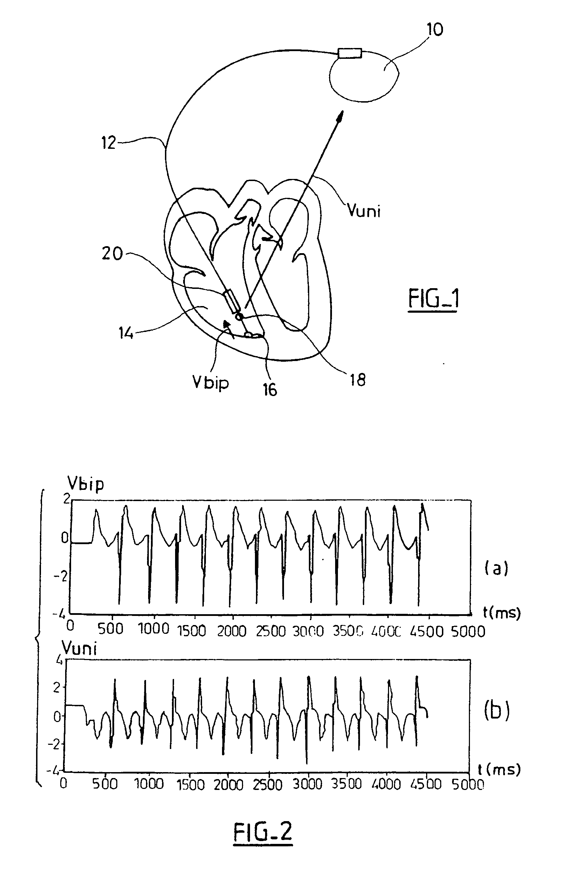

La Figure 1 illustre une configuration de stimulation de type "simple chambre" dans laquelle un générateur d'impulsions 10 est associé à une sonde 12 implantée dans le ventricule droit 14. La tête de sonde comporte deux électrodes, à savoir une électrode distale 16 et une électrode proximale 18, ce qui permet de recueillir, dans cette configuration la plus simple, un premier électrogramme *Vbip* correspondant à la différence de potentiel mesurée entre l'électrode distale 16 et l'électrode proximale 18, et d'autre part un second électrogramme *Vuni,* mesuré par la différence de potentiel entre l'une des électrodes, par exemple l'électrode proximale 18, et le boîtier métallique du générateur 10.

Dans le cas d'un défibrillateur, le signal bipolaire *Vbip* peut être également recueilli entre l'électrode distale 16 et le bobinage ou coil ventriculaire formant électrode de défibrillation 20, et le signal unipolaire *Vuni* peut être recueilli entre le boîtier métallique du générateur 10 et ce bobinage ventriculaire 20, ou encore entre l'électrode proximale 18 et le boîtier 10 lorsque le signal bipolaire *Vbip* est recueilli entre l'électrode distale 16 et le bobinage ventriculaire 20.

On notera que l'invention peut être appliquée également au filtrage des signaux recueillis dans l'oreillette par une électrode appropriée, ou de façon générale à tout recueil de signaux par des électrodes de détection de signaux de dépolarisation cardiaque, notamment dans le cas des dispositifs multisite.

De façon générale, on parlera de "cavité", ce terme pouvant s'appliquer indifféremment à l'oreillette ou au ventricule, et aux cavités droites aussi bien qu'aux cavités gauches.

Comme cela a été indiqué plus haut, la technique d'analyse de l'invention consiste, pour chaque battement cardiaque, à opérer une classification entre cycle non bruité valide (onde de dépolarisation d'origine cardiaque) et cycle bruité invalide (artéfact d'origine extracardiaque), à partir de signaux d'électrogramme EGM issus d'une même cavité, typiquement le ventricule droit, et recueillis sur deux voies distinctes et analysés en deux dimensions.

La Figure 2 illustre un exemple de tracés d'électrogrammes *Vbip* et *Vuni* observés respectivement sur la voie bipolaire ventriculaire (Fig. 2a) et sur la voie unipolaire ventriculaire (Fig. 2b) de la configuration illustrée Figure 1.

Il n'est toutefois pas indispensable d'analyser la totalité du cycle, l'analyse d'une fraction significative de ce cycle (typiquement, celle centrée autour du complexe QRS) étant en général suffisante pour permettre de discriminer entre cycle bruité invalide et cycle non bruité valide.

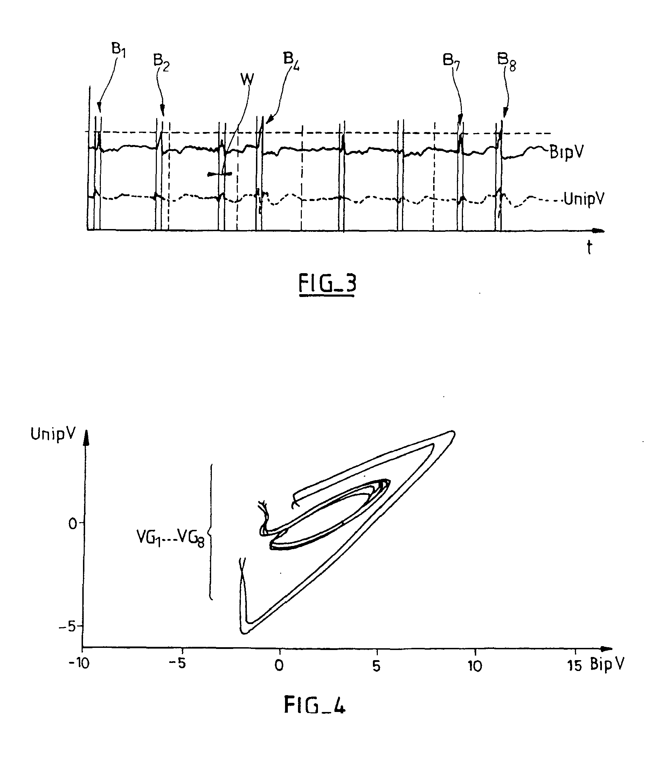

Plus précisément, comme illustré Figure 3, après chaque détection d'un pic de dépolarisation sur le signal bipolaire *Vbip* (correspondant à une onde R détectée), le battement correspondant est isolé par une fenêtre fixe W de quelques dizaines de millisecondes centrée sur ce pic de dépolarisation, par exemple une fenêtre de largeur W = 80 ms correspondant à 80 points pour une fréquence d'échantillonnage de 1000 Hz.

Les battements successifs $B_1$ ... $B_8$ sont enregistrés simultanément sur la voie bipolaire ventriculaire (*Vbip*) et la voie unipolaire ventriculaire *(Vuni),* et la fraction de chacun de ces battements comprise à l'intérieur de la fenêtre W est alors représentée sous forme d'une caractéristique considérée dans le plan constitué de la voie bipolaire *Vbip* en abscisse et de la voie unipolaire *Vuni* en ordonnée. Cette caractéristique est dénommée "boucle cardiaque" ou "vectogramme" VGM (le vectogramme VGM devant être distingué, comme indiqué plus haut, du "vectocardiogramme" VCG, qui est obtenu à partir de signaux d'électrocardiogramme ECG externes, et non de signaux EGM qui sont des signaux intracardiaques).

Cette boucle VGM constitue donc une représentation d'un battement cardiaque complet dans un espace non temporel.

Les VGM correspondants $V_1$ ... $V_8$ sont illustrés Figure 4. On notera que dans ce cas les VGM ne sont pas des boucles fermées, dans la mesure où ils ne correspondent qu'à une partie de la boucle cardiaque complète, à savoir le complexe QRS isolé à l'intérieur de la fenêtre W.

On notera que les EGM de la Figure 3 et les VGM de la Figure 4 correspondent à un échantillonnage à une fréquence de 1000 Hz, représentant environ 80 points d'échantillonnage pour chaque VGM, donc pour chaque cycle analysé.

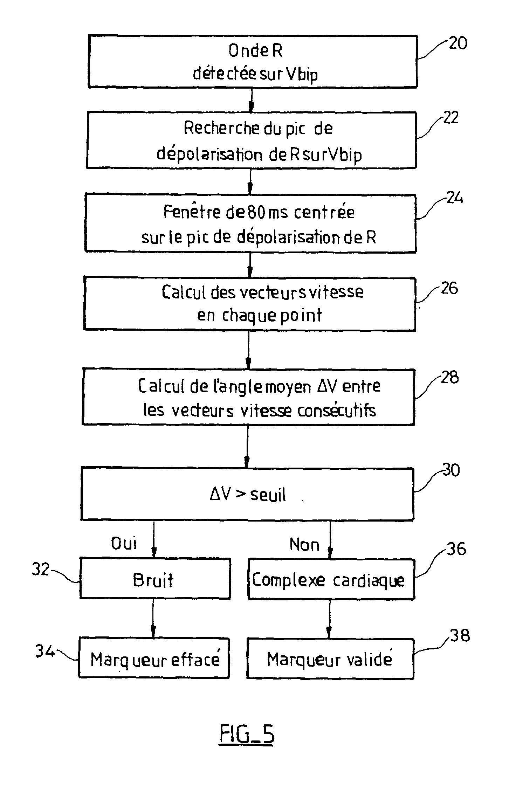

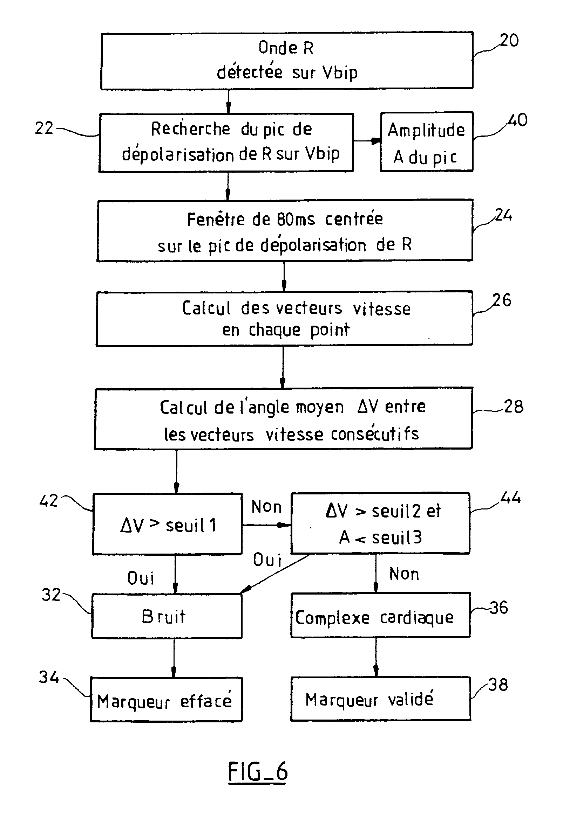

L'invention se révèle toutefois efficace même avec un échantillonnage à fréquence beaucoup plus faible, par exemple à 128 Hz, donnant environ 10 points seulement par VGM, comme dans le cas des divers cycles représentés sur les Figures 5 et 6.

L'invention consiste, essentiellement, pour chaque cycle donc pour chaque VGM ainsi constitué, à opérer une discrimination entre cycle bruité invalide et cycle non bruité valide par une analyse du VGM correspondant. Cette analyse est opérée de la manière illustrée par l'organigramme de la Figure 5.

Les premières étapes consistent à opérer le fenêtrage expliqué plus haut en référence à la Figure 3, par détection de l'onde R sur la voie bipolaire (étape 20), recherche du pic de dépolarisation R de cette onde (étape 22) et sélection de la partie du signal comprise dans une fenêtre de 80 ms centrée sur ce pic R (étape 24).

L'étape suivante consiste à décrire le vectogramme par un paramètre lié à la morphologie intrinsèque du VGM courant. L'analyse de ce paramètre descripteur morphologique permettra de déterminer ensuite si le battement courant est d'origine cardiaque, ou s'il s'agit de bruit.

Ce paramètre descripteur est par exemple l'angle moyen que forment les vecteurs vitesse consécutifs le long du parcours de la boucle du VGM.

Le choix de ce descripteur est basé sur l'hypothèse que pour un complexe cardiaque non bruité (signal dont l'origine est une onde de dépolarisation cardiaque), deux vecteurs vitesse consécutifs ne doivent pas trop changer de direction. En effet, dans le cas d'un battement cardiaque non bruité, il existe entre les signaux détectés sur les deux voies une corrélation qui fait que le VGM progresse de façon régulière. Inversement, dans le cas d'un cycle bruité (artefact d'origine extra-cardiaque), le VGM progresse de façon erratique, de sorte que deux vecteurs vitesse consécutifs peuvent être dirigés dans des directions très différentes, voire même opposées.

Le choix du vecteur vitesse n'est toutefois pas limitatif, et d'autres descripteurs peuvent être utilisés - en variante ou en complément - pour opérer la discrimination entre battement d'origine cardiaque et bruit, par exemple la variance de l'angle moyen entre les vecteurs vitesse consécutifs, ou plus généralement encore, tout descripteur représentatif d'une progression régulière des points consécutifs du VGM.

Le vecteur vitesse $\bar{v}(t)$ est calculé (étape 26) en chaque point *t* à partir d'un filtre discret qui approxime les dérivées premières $V'_{bip}(t)$ et $V'_{uni}(t)$ (sur 4 points pour une fréquence d'échantillonnage de 1000 Hz et sur 2 points pour 128 Hz) :

$$V'_{bip}(t) = \frac{V_{bip}(t+2) + 2V_{bip}(t+1) - 2V_{bip}(t-1) - V_{bip}(t-2)}{6}$$

$$V'_{uni}(t) = \frac{V_{uni}(t+2) + 2V_{uni}(t+1) - 2V_{uni}(t-1) - V_{uni}(t-2)}{6}$$

$$\vec{v}(t) = \left(V'_{bip}(t), V'_{uni}(t)\right)$$

Pour le calcul (étape 28) du descripteur $\Delta V$ correspondant à l'angle moyen entre deux vecteurs vitesse consécutifs, on peut avantageusement utiliser le cosinus de chaque angle. En effet, avec des vecteurs unitaires, le cosinus de l'angle que forment deux vecteurs (*a,b*) et (*c,d*) est égal au produit scalaire *a.c+b.d* de ces vecteurs, ce qui simplifie grandement les calculs. $\Delta V$ s'exprime par :

$$\Delta V = \left| mean\left( \cos\left(angle(\vec{v}(1), \vec{v}(2))\right), \cos\left(angle(\vec{v}(2), \vec{v}(3))\right), ..., \cos\left(angle(\vec{v}(n-1), \vec{v}(n))\right)\right) - 1 \right|$$

et prend des valeurs comprises entre 0 (pour un angle de 0°) et 2 (pour un angle de 180°).

Le descripteur $\Delta V$ ainsi calculé est ensuite comparé à un seuil donné, par exemple un seuil de 0,9 (étape 30) :

- si $\Delta V$ est supérieur à ce seuil, alors le VGM ne représente pas un complexe cardiaque, le cycle est considéré comme un cycle bruité invalide (étape 32) et il est effacé de la chaîne des marqueurs (étape 34) ;
- si, en revanche, $\Delta V$ est inférieur ou égal au seuil, alors le cycle est considéré comme un cycle non bruité valide, d'origine cardiaque (étape 36) et le marqueur correspondant est validé (étape 38).

L'organigramme de la Figure 6 illustre une variante perfectionnée de l'organigramme de la Figure 5, permettant d'éviter toute sous-détection notamment dans le cas de fibrillations ventriculaires.

À cet effet, après calcul du descripteur $\Delta V$ (étapes 20-28, identiques à celles de la figure 5) $\Delta V$ est comparé à deux seuils différents, dont l'un sera fonction de l'amplitude A du pic de dépolarisation, mémorisée (étape 40) au moment de la recherche de ce pic sur le signal de la voie bipolaire (étape 22).

Comme dans le cas précédent, le descripteur $\Delta V$ sera comparé à un premier seuil *Seuil1* indépendant de l'amplitude du pic de dépolarisation, mais ce seuil sera plus élevé, par exemple supérieur à 1. Si $\Delta V$ est supérieur à *Seuil1* (étape 42), alors comme dans le cas précédent le cycle est considéré comme un cycle bruité invalide (étapes 32 et 34).

Dans le cas contraire, $\Delta V$ est comparé à un deuxième seuil *Seuil2* inférieur à *Seuil1*, par exemple *Seuil2* = 0,8, et ce seuil ne sera appliqué que si l'amplitude A du pic de dépolarisation est suffisamment faible (par *exemple A < Seuil3* = 3 mV). Si ces deux conditions sont vérifiées, alors le cycle est considéré comme un cycle bruité invalide (étapes 32-34), dans le cas contraire, le cycle est considéré comme un cycle non bruité valide, représentatif d'un complexe cardiaque (étapes 36-38).

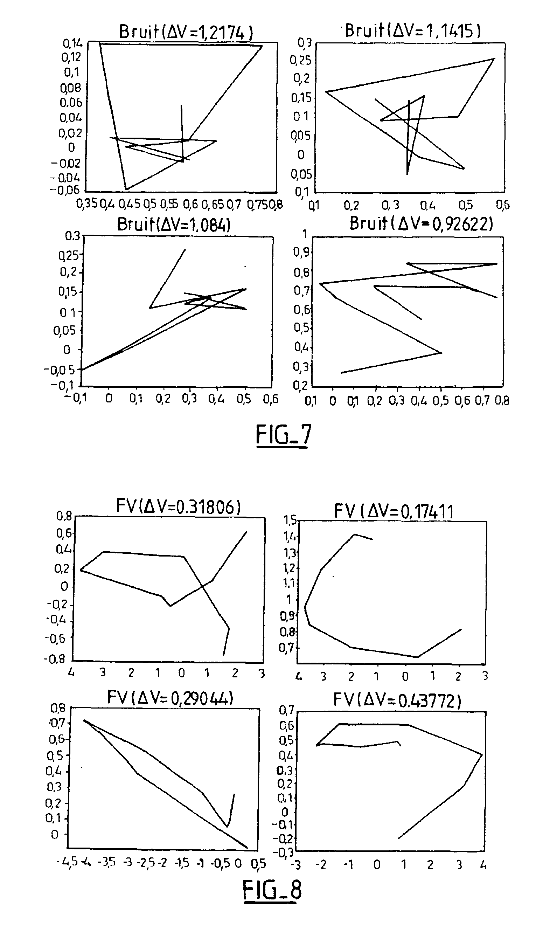

Les Figures 7 et 8 illustrent divers exemples de vectogrammes analysés par la technique de l'invention et classés respectivement comme bruit et comme signaux valides de fibrillation ventriculaire.

On a également indiqué sur ces figures les valeurs calculées du paramètre descripteur $\Delta V$.

Sur les cycles d'origine non cardiaque (ceux de la Figure 7), on note des changements de direction nombreux et très brusques, typiques d'un signal de bruit, d'origine non cardiaque.

En revanche, dans le cas de cycles bruités d'origine cardiaque (ceux de la Figure 8), les transitions sont beaucoup plus progressives, et ce pour la totalité ou la quasi-totalité des points du VG. On peut certes observer une ou deux variations localement brusques, mais à la différence des signaux de bruit celles-ci sont toujours en nombre très faible, ce qui conduit à une valeur relativement faible pour l'angle moyen, et donc au final pour le descripteur $\Delta V$.

Diverses actions peuvent être entreprises à la suite de la classification entre cycles valides et cycles invalides, notamment :

- effacement des marqueurs correspondant aux cycles invalides bruités ;
- modification éventuelle de la sensibilité des circuits de détection ;
- inhibition d'une thérapie ;
- modification du label d'identification des épisodes ;
- effacement de la mémoire des épisodes de bruit, notamment pour les épisodes non soutenus.

Incidemment, on peut remarquer que si le défibrillateur met déjà en oeuvre un algorithme de discrimination des arythmies TV/TSV par la technique décrite dans le EP 2 105 843 A1 précité, le VGM aura déjà été constitué et analysé, et les vecteurs vitesse calculés, de sorte que la technique d'élimination du bruit selon la présente invention pourra être mise en oeuvre avec un coût très réduit en termes de ressources logicielles.

**Revendications**

**1.**   Un dispositif médical actif (10, 12), comprenant :

- des moyens de recueil de l'activité électrique du coeur sur une série de cycles cardiaques, comprenant des moyens pour produire au moins deux composantes temporelles distinctes ($Vbip$, $Vuni$) à partir d'au moins deux signaux EGM d'électrogramme endocavitaire provenant d'une même cavité du coeur et recueillis concurremment sur des voies respectives distinctes ; et
- des moyens d'analyse et de filtrage de l'activité électrique recueillie, aptes à discriminer, pour chaque cycle cardiaque analysé, entre cycle bruité invalide et cycle non bruité valide,

dispositif **caractérisé en ce que** les moyens d'analyse et de filtrage comprennent :

- des moyens pour déterminer une caractéristique 2D non-temporelle (VGM) représentative dudit cycle cardiaque analysé, à partir des variations d'une desdites composantes temporelles ($Vuni$) en fonction d'une l'autre ($Vbip$) ;
- des moyens d'analyse morphologique de ladite caractéristique 2D, aptes à délivrer au moins un paramètre descripteur intrinsèque de cette caractéristique ; et
- des moyens classifieurs, aptes à classer ledit cycle cardiaque analysé comme cycle bruité invalide ou bien comme cycle non bruité valide, en fonction dudit au moins un paramètre descripteur.

**2.**   Le dispositif de la revendication 1, dans lequel lesdits signaux EGM recueillis concurremment sur des voies respectives distinctes comprennent :

- un signal de composante unipolaire *(Vuni)* recueilli entre le boîtier du dispositif et une électrode proximale ou distale, et
- un signal de composante bipolaire ($Vbip$) recueilli entre une électrode proximale et une électrode distale.

**3.**   Le dispositif de la revendication 1, dans lequel les moyens pour déterminer la caractéristique 2D sont des moyens aptes à déterminer cette caractéristique à partir des variations desdites composantes temporelles sur une fraction du cycle cardiaque analysé, considérée dans une fenêtre temporelle incluant le complexe QRS de ce cycle cardiaque.

**4.**   Le dispositif de la revendication 1, dans lequel ledit paramètre descripteur est un paramètre fonction du vecteur vitesse de la caractéristique 2D, considéré en une pluralité de points (P).

**5.**   Le dispositif de la revendication 4, dans lequel ledit paramètre descripteur est fonction des variations de la direction du vecteur vitesse entre des points consécutifs donnés de la caractéristique 2D.

**6.**   Le dispositif de la revendication 5, dans lequel ledit paramètre descripteur est fonction de l'angle moyen que forment deux vecteurs vitesse consécutifs de la caractéristique 2D.

**7.**   Le dispositif de la revendication 6, dans lequel les moyens classifieurs comprennent des moyens pour :

- comparer l'angle moyen à un premier seuil donné, et
- classer le cycle cardiaque à analyser comme cycle bruité invalide si l'angle moyen est supérieur à ce premier seuil.

8. Le dispositif de la revendication 7, dans lequel les moyens classifieurs comprennent des moyens pour, si l'angle moyen est inférieur audit premier seuil :

- comparer l'angle moyen à un deuxième seuil donné, inférieur au premier seuil,
- comparer l'amplitude du pic de dépolarisation du cycle cardiaque analysé à un troisième seuil, et
- classer le cycle cardiaque à analyser comme cycle bruité invalide si l'angle moyen est supérieur au deuxième seuil et l'amplitude du pic de dépolarisation inférieure au troisième seuil, et comme cycle non bruité dans le cas contraire.

9. Le dispositif de la revendication 4, dans lequel ledit paramètre descripteur est fonction de la variance de la norme du vecteur vitesse entre des points consécutifs donnés de la caractéristique 2D.

10. Le dispositif de la revendication 1, comprenant en outre des moyens opérant en réponse aux moyens classifieurs et aptes à :

- effacer un marqueur lié au cycle cardiaque analysé, et/ou
- attribuer un marqueur spécifique au cycle cardiaque analysé, et/ou
- inhiber la mémorisation du cycle cardiaque analysé, et/ou
- modifier la sensibilité des moyens de recueil, et/ou
- inhiber la délivrance d'une thérapie par le dispositif,

lorsque le cycle analysé est classé comme cycle bruité invalide par les moyens classifieurs.

## Claims

1. Active medical device (10, 12), comprising:

- means for acquiring the electrical activity of the heart over a series of cardiac cycles, comprising means for producing at least two separate temporal components (*Vbip, Vuni*) from at least two endocavity electrogram EGM signals coming from the same cavity of the heart and aquired concurrently on separate respective channels; and
- means for analyzing and filtering the acquired electrical activity, which, for each analyzed cardiac cycle, are capable of discriminating between an invalid cycle affected by noise and a valid cycle not affected by noise,

which device is **characterized in that** the analysis and filtering means comprise:

- means for determining a non-temporal 2D characteristic (VGM), representative of the said analyzed cardiac cycle, from the variations of one of the said temporal components (*Vuni*) as a function of one another (*Vbip*);
- means for morphological analysis of the said 2D characteristic, which are capable of delivering at least one intrinsic descriptor parameter of this characteristic; and
- classifier means capable of classifying the said analyzed cardiac cycle as an invalid cycle affected by noise or as a valid cycle not affected by noise, as a function of the said at least one descriptor parameter.

2. Device according to Claim 1, wherein the said EGM signals acquired concurrently on separate respective channels comprise:

- a unipolar component signal (*Vuni*) acquired between the housing of the device and a proximal or distal electrode, and
- a bipolar component signal (*Vbip*) acquired between a proximal electrode and a distal electrode.

3. Device according to Claim 1, wherein the means for determining the 2D characteristic are means capable of determining this characteristic from the variations of the said temporal components over a fraction of the analyzed cardiac cycle, considered as a temporal window including the QRS complex of this cardiac cycle.

4. Device according to Claim 1, wherein the said descriptor parameter is a parameter which is a function of the velocity vector of the 2D characteristic, considered at a plurality of points (P).

5. Device according to Claim 4, wherein the said descriptor parameter is a function of the variations of the direction of the velocity vector between given consecutive points of the 2D characteristic.

6. Device according to Claim 5, wherein the said descriptor parameter is a function of the average angle formed by two consecutive velocity vectors of the 2D characteristic.

7. Device according to Claim 6, wherein the classifier means comprise means for:

- comparing the average angle with a first given threshold, and
- classifying the cardiac cycle to be analyzed as an invalid cycle affected by noise if the average angle is greater than this first threshold.

8. Device according to Claim 7, wherein the classifier means comprise means for, if the average angle is less than the said first threshold:

- comparing the average angle with a given second threshold, which is less than the first threshold,
- comparing the amplitude of the depolarization peak of the analyzed cardiac cycle with a third threshold, and
- classifying the cardiac cycle to be analyzed as an invalid cycle affected by noise if the average angle is greater than the second threshold and the amplitude of the depolarization peak is less than the third threshold, and as a cycle not affected by noise in the converse case.

9. Device according to Claim 4, wherein the said descriptor parameter is a function of the variance of the norm of the velocity vector between given consecutive points of the 2D characteristic.

10. Device according to Claim 1, furthermore comprising means operating in response to the classifier means and capable of:

- erasing a marker linked to the analyzed cardiac cycle, and/or
- assigning a specific marker to the analyzed cardiac cycle, and/or
- preventing storage of the analyzed cardiac cycle in memory, and/or
- modifying the sensitivity of the acquisition means, and/or
- preventing the delivery of a therapy by the device,

when the analyzed cycle is classified by the classifier means as an invalid cycle affected by noise.


**Patentansprüche**

1. Aktive medizinische Vorrichtung (10, 12), die Folgendes umfasst:

- Mittel zum Erfassen der elektrischen Aktivität des Herzens in einer Reihe von Herzzyklen, mit Mitteln, um anhand von wenigstens zwei EGM-Signalen eines endokavitären Elektrogramms, die vom selben Hohlraum des Herzens stammen und konkurrent auf jeweils verschiedenen Wegen erfasst werden, wenigstens zwei verschiedene zeitliche Komponenten (Vbip, Vuni) zu erzeugen; und
- Mittel zum Analysieren und Filtern der erfassten elektrischen Aktivität, die für jeden analysierten Herzzyklus zwischen einem ungültigen verrauschten Zyklus und einem gültigen nicht verrauschten Zyklus unterscheiden können,

wobei die Vorrichtung **dadurch gekennzeichnet ist, dass** die Analyse- und Filterungsmittel Folgendes umfassen:

- Mittel zum Bestimmen einer nicht zeitlichen 2D-Charakteristik (VGM), die den analysierten Herzzyklus repräsentiert, anhand von Veränderungen einer der zeitlichen Komponenten (Vuni) als Funktion der jeweils anderen (Vbip);
- Mittel zur morphologischen Analyse dieser 2D-Charakteristik, die wenigstens einen intrinsischen deskriptiven Parameter dieser Charakteristik liefern können; und

- Klassifizierungsmittel, die den analysierten Herzzyklus als Funktion des wenigstens einen beschreibenden Parameters als ungültigen verrauschten Zyklus oder aber als gültigen nicht verrauschten Zyklus klassifizieren können.

**2.** Vorrichtung nach Anspruch 1, wobei die EGM-Signale, die auf den jeweils verschiedenen Wegen konkurrent erfasst werden, Folgendes umfassen:

- ein Signal (Vuni) einer unipolaren Komponente, das zwischen dem Gehäuse der Vorrichtung und einer proximalen oder distalen Elektrode erfasst wird, und
- ein Signal (Vbip) einer bipolaren Komponente, das zwischen einer proximalen Elektrode und einer distalen Elektrode erfasst wird.

**3.** Vorrichtung nach Anspruch 1, wobei die Mittel zum Bestimmen der 2D-Charakteristik Mittel sind, die diese Charakteristik anhand von Veränderungen der zeitlichen Komponenten in einem Bruchteil des analysierten Herzzyklus, der in einem Zeitfenster betrachtet wird, das den QRS-Komplex dieses Herzzyklus enthält, bestimmen können.

**4.** Vorrichtung nach Anspruch 1, wobei der beschreibende Parameter ein Parameter ist, der von dem Geschwindigkeitsvektor der 2D-Charakteristik, der an mehreren Punkten (P) betrachtet wird, abhängt.

**5.** Vorrichtung nach Anspruch 4, wobei der beschreibende Parameter eine Funktion von Veränderungen der Richtung des Geschwindigkeitsvektors zwischen gegebenen aufeinanderfolgenden Punkten der 2D-Charakteristik ist.

**6.** Vorrichtung nach Anspruch 5, wobei der beschreibende Parameter eine Funktion des mittleren Winkels ist, den zwei aufeinanderfolgende Geschwindigkeitsvektoren der 2D-Charakteristik bilden.

**7.** Vorrichtung nach Anspruch 6, wobei die Klassifikationsmittel Mittel umfassen, um:

- den mittleren Winkel mit einem ersten gegebenen Schwellenwert zu vergleichen und
- den zu analysierenden Herzzyklus als ungültigen verrauschten Zyklus zu klassifizieren, wenn der mittlere Winkel größer als dieser erste Schwellenwert ist.

**8.** Vorrichtung nach Anspruch 7, wobei die Klassifikationsmittel Mittel umfassen, um dann, wenn der mittlere Winkel kleiner als der erste Schwellenwert ist:

- den mittleren Winkel mit einem zweiten gegebenen Schwellenwert, der kleiner als der erste Schwellenwert ist, zu vergleichen,
- die Amplitude der Depolarisationsspitze des analysierten Herzzyklus mit einem dritten Schwellenwert zu vergleichen und
- den zu analysierenden Herzzyklus als ungültigen verrauschten Zyklus zu klassifizieren, wenn der mittlere Winkel größer als der zweite Schwellenwert ist und die Amplitude der Depolarisationsspitze kleiner als der dritte Schwellenwert ist, und andernfalls als nicht verrauschten Zyklus zu klassifizieren.

**9.** Vorrichtung nach Anspruch 4, wobei der beschreibende Parameter eine Funktion der Varianz des Betrags des Geschwindigkeitsvektors zwischen gegebenen aufeinanderfolgenden Punkten der 2D-Charakteristik ist.

**10.** Vorrichtung nach Anspruch 1, die außerdem Mittel umfasst, die in Reaktion auf die Klassifikationsmittel arbeiten und dazu ausgelegt sind,

- einen mit dem analysierten Herzzyklus in Beziehung stehenden Markierer zu löschen und/oder
- einen für den analysierten Herzzyklus spezifischen Markierer hinzuzufügen und/oder
- das Speichern des analysierten Herzzyklus zu verhindern und/oder
- die Empfindlichkeit der Erfassungsmittel zu modifizieren und/oder
- die Verabreichung einer Therapie durch die Vorrichtung zu verhindern,

wenn der analysierte Zyklus durch die Klassifikationsmittel als ungültiger verrauschter Zyklus klassifiziert wird.

FIG_1

FIG_2

FIG_3

FIG_4

```
                    ┌─────────────────────────┐  ╱20
                    │   Onde R                 │
                    │   détectée sur Vbip      │
                    └───────────┬─────────────┘
                                │
                    ┌───────────▼─────────────┐  ╱22
                    │   Recherche du pic de    │
                    │  dépolarisation de R sur Vbip │
                    └───────────┬─────────────┘
                                │
                    ┌───────────▼─────────────┐  ╱24
                    │  Fenêtre de 80 ms centrée │
                    │  sur le pic de dépolarisation de R │
                    └───────────┬─────────────┘
                                │
                    ┌───────────▼─────────────┐  ╱26
                    │  Calcul des vecteurs vitesse │
                    │  en chaque point         │
                    └───────────┬─────────────┘
                                │
                    ┌───────────▼─────────────┐  ╱28
                    │ Calcul de l'angle moyen ΔV entre │
                    │ les vecteurs vitesse consécutifs │
                    └───────────┬─────────────┘
                                │
                    ┌───────────▼─────────────┐  ╱30
                    │        ΔV > seuil        │
                    └────┬────────────────┬───┘
                     Oui │            Non │
        32 ┌─────────────▼──┐     ┌──────▼──────────────┐  ╱36
           │     Bruit      │     │  Complexe cardiaque │
           └───────┬────────┘     └───────┬─────────────┘
                   │                      │
        34 ┌───────▼────────┐     ┌───────▼─────────────┐  ╱38
           │ Marqueur effacé │    │  Marqueur validé    │
           └────────────────┘     └─────────────────────┘
```

FIG_5

```
                    ┌─────────────────────────┐
                    │        Onde R           │  ⟋20
                    │   détectée sur Vbip     │
                    └───────────┬─────────────┘
                                │
        22 ┌────────────────────┴──────────┐    ┌──────────────┐
           │   Recherche du pic de         │───▶│  Amplitude   │ ⟋40
           │ dépolarisation de R sur Vbip  │    │   A du pic   │
           └───────────────┬───────────────┘    └──────────────┘
                           │
           ┌───────────────┴───────────────┐  ⟋24
           │   Fenêtre de 80ms centrée      │
           │  sur le pic de dépolarisation de R │
           └───────────────┬───────────────┘
                           │
           ┌───────────────┴───────────────┐  ⟋26
           │   Calcul des vecteurs vitesse  │
           │        en chaque point         │
           └───────────────┬───────────────┘
                           │
           ┌───────────────┴───────────────┐  ⟋28
           │ Calcul de l'angle moyen ΔV entre│
           │  les vecteurs vitesse consécutifs│
           └───────────────┬───────────────┘
```

42 ΔV > seuil 1 — Non → 44 ΔV > seuil2 et A < seuil3

Oui ↓          Oui ↙          Non ↓

32 Bruit          36 Complexe cardiaque

34 Marqueur effacé          38 Marqueur validé

FIG_6

FIG_7

FIG_8

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 6584351 B1
- US 20060235476 A1
- US 7215993 B2
- EP 2105843 A1

**Littérature non-brevet citée dans la description**

- **Schulte B et al.** Inappropriate Arrhythmia Detection in Implantable Defibrillator Therapy Due to Oversensing of Diaphragmatic Myopotentials. *J Interv Card Electrophysiol,* 2001, vol. 5 (4), 487-93
- **Occhetta E et al.** Inappropriate Implantable Cardioverter-Defibrillator Discharges Unrelated to Supraventricular Tachyarrhythmias. *Europace,* 2006, vol. 8 (10), 863-9